# EUROPÄISCHE PATENTSCHRIFT

(11) **EP 0 325 141 B1**
(45) Veröffentlichungstag und Bekanntmachung des Hinweises auf die Patenterteilung: **10.11.1993**
(21) Anmeldenummer: 89100327.9
(22) Anmeldetag: 10.01.1989
(51) Int. Cl.: C07C 33/20, C07C 29/132

(54) **Verfahren zur Herstellung von Phenylethanolen**
Process for the preparation of phenyl ethanols
Procédé de préparation de phényléthanols

(30) Priorität: 16.01.1988 DE 3801106
(43) Veröffentlichungstag der Anmeldung: 26.07.1989
(73) Patentinhaber: BASF Aktiengesellschaft, 67063 Ludwigshafen (DE)
(72) Erfinder: Hoelderich, Wolfgang, Dr., D-6710 Frankenthal (DE); Goetz, Norbert, Dr., D-6520 Worms 1 (DE); Hupfer, Leopold, Dr., D-6701 Friedelsheim (DE)

(56) Entgegenhaltungen:
- EP-A- 0 096 798
- DE-A- 3 239 611
- US-A- 4 014 945

## Beschreibung

Die Erfindung betrifft ein Verfahren zur Herstellung von Phenylethanolen durch Umsetzung von Styroloxiden mit Wasserstoff in Gegenwart von Zeolithen und/oder Phosphaten, die mit Hydriermetallen dotiert sind.

Phenylethanole sind bedeutende Riechstoffe, die in der Parfümerie- und Kosmetikindustrie breite Anwendung finden. Technisch wird Phenylethanol aus Benzol und Ethylenoxid in Gegenwart eines Lewis-Katalysators wie AlCl₃ hergestellt. Nachteilig hierbei sind Probleme mit auftretender Korrosion, Katalysatorabtrennung und Abwasserverschmutzung. Auch ist die Ausbeute noch verbesserungswürdig.

Es ist auch bekannt, daß man Phenylethanole durch Hydrierung von Styroloxiden mit Raney-Ni bzw. Pd auf Aktivkohle in basischem Medium (DE 26 41 821) oder an Raney-Co-Pd-Katalysator (JP 77 108 940) oder an Rhodium-Komplexen (J.Org.Chem. 46 (1981), 2287-2290) oder mit Diboran zusammen mit H₂O₂ (Aust.J.Chem. 30 (1977), 141-150) enthält. Hierbei ist die Abtrennung der Suspensionskatalysatoren bzw. Homogenkatalysatoren oder die Verwendung leichtentzündlicher Reagentien von Nachteil.

In DE-PS 2 206 805 wird die Umsetzung von Styroloxid zu Phenylethanol wird bei 20 bis 120°C an einem Pd-Katalysator auf SiO₂- bzw. Al₂O₃-Träger beschrieben. Nachteilig ist, daß die Umsetzung in einem organischen Lösungsmittel wie n-Hexan und bei hoben Drücken von 100 bis 300 atm durchgeführt werden muß.

Die Nachteile der bisher bekannten Verfahren zur Herstellung von Phenylethanolen aus Styroloxiden sind so schwerwiegend, daß sich dieses Verfahren nicht gegen die Herstellung aus Benzol und Ethylenoxid technisch durchsetzen konnte.

Es wurde gefunden, daß man Phenylethanole der Formel (I) 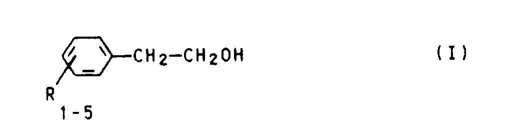
wobei R Wasserstoff, Alkyl- mit 1 bis 4 Kohlenstoffatomen, Alkoxy- mit 1 bis 4 Kohlenstoffatomen, Fluor-, Trifluormethyl- und/oder Trifluormethoxy-Reste bedeuten, erhält, wenn man Epoxide der Formel (II) 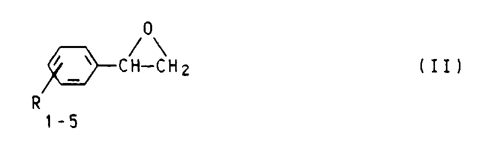
wobei R obige Bedeutung besitzt, in Gegenwart von Wasserstoff an Zeolithen in der aciden H-Form und/oder Phosphaten, die mit Hydriermetallen dotiert sind, als Katalysatoren umsetzt.

Durch das erfindungsgemäße Verfahren werden die erwähnten Nachteile der bisherigen Verfahrensweisen vermieden und die an die Katalysatoren gestellten Anforderungen bezüglich hoher Selektivität bei hohem Umsatz sowie hohe Standzeit und Regenerierbarkeit, erfüllt. Weitere Vorteile des erfindungsgemäßen Verfahrens sind: Vollständiger Umsatz, keine Trennprobleme, lange Standzeiten, hohe Selektivitäten - auch für fluorhaltige Verbindungen - einfache Isolierung der Endprodukte, leichte Regenerierbarkeit der Katalysatoren bei eventuell auftretender Verkokung.

Als Ausgangsstoffe kann man z.B. Styroloxid, p-Fluorstyroloxid, 2, 4-Di-fluorstyroloxid, 3,4-Difluorstyroloxid, 2,4,5-Trifluorstyroloxid, o-, m-, p-Trifluormethylstyroloxid, o-, m-, p-Methylstyroloxid, o-, m-, p-Methoxystyroloxid, 2,3,4,5-Tetrafluorstyroloxid, p-Trifluormethoxystyroloxid, 2-Fluor-4-trifluormethylstyroloxid, 2-Fluor-4-trifluormethoxy-styroloxid und 2-Methyl-4-fluorstyroloxid verwenden.

Als Katalysatoren für das erfindungsgemäße Verfahren werden acide zeolithische Katalysatoren eingesetzt. Zeolithe sind kristalline Aluminosilikate, die eine hochgeordnete Struktur mit einem starren dreidimensionalen Netzwerk von SiO₄- und AlO₄-Tetraedern besitzen, die durch gemeinsame Sauerstoffatome verbunden sind. Das Verhältnis der Si- und Al-Atome zu Sauerstoff beträgt 1:2 (siehe Ullmanns Encyclopädie d. techn. Chemie, 4. Auflage, Band 24, Seite 575 (1983)). Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Für das erfindungsgemäße Verfahren kommen insbesondere in Betracht Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe. In diese Gruppe von Zeolithen gehören auch die sogenannten "ultrastabilen" Zeolithe des Faujasittyps, d.h. dealuminierte Zeolithe. Verfahren zur Herstellung solcher Zeolithe sind beschrieben in "Catalysis by Zeolites" Band 5 aus "Studies in Surface Science and Catalysis" ed. B. Imelik et.al. Elsevier Scientific Publishing Comp, 1980, S. 203 und "Crystal Structures of Ultra-stable Faujasites" Advances in Chemistry Series Nr. 101, American Chemical Society Washington, DC, S. 226 ff (1971) und in US-PS 4 512 961.

Besonders vorteilhaft sind Zeolithe vom Pentasiltyp. Diese haben als Grundbaustein einen aus SiO₄-Tetraedern aufgebauten Fünfring gemeinsam. Sie sind durch ein hohes SiO₂/Al₂O₃-Verhältnis gekennzeichnet sowie durch Porengroßen, die zwischen denen der Zeolithe vom Typ A und denen vom Typ X oder Y liegen (vgl. Ullmanns Encyclopädie d. techn. Chemie, 4. Auflage, Band 24, 1983).

Diese Zeolithe können unterschiedliche chemische Zusammensetzung aufweisen. Es handelt sich hierbei um Alumino-, Boro-, Eisen-, Beryllium-> Gallium-, Chrom-, Arsen-, Antimon- und Wismutsilikatzeolithode oder deren Gemische sowie Alumino-, Boro-, Gallium- und Eisengermanatzeolithe oder deren Gemische. Insbesondere eignen sich die Alumino-, Boro- und Eisensilikatzeolithe des Pentasiltyps für das erfindungsgemäße Verfahren, Der Aluminosilikatzeolith wird z.B. aus einer Aluminiumverbindung, vorzugsweise Al(OH)₃ oder Al₂(SO₄)₃ und einer Siliciumkomponente, vorzugsweise hochdispersem Siliciumdioxid in wäßriger Aminlösung, insbesondere in Polyaminen wie 1 ,6-Hexandiamin- oder 1 ,3-Propandiamin- oder Triethylentetramin-Lösung mit oder insbesondere ohne Alkali- oder Erdalkalizusatz bei 100 bis 220°C unter autogenem Druck hergestellt. Auch gehören hierzu die isotaktiscflen Zeolithe nach EP 34 727 und EP 46 504. Die erhaltenen Aluminosilikatzeolithe enthalten je nach Wahl der Einsatzstoffmengen ein SiO₂/Al₂O₃)-Verhältnis von 10 bis 40.000. Auch lassen sich derartige Aluminosilikatzeolithe in etherischem Medium wie Diethylenglykoldimethylether, in alkoholischem Medium wie Methanol bzw. 1 ,4-Butandiol oder in Wasser synthetisieren.

Der Borosilikatzeolith wird z.B. bei 90 bis 200°C unter autogenem Druck synthetisiert, indem man eine Borverbindung, z.8. H₃BO₃, mit einer Siliciumverbindung, vorzugsweise hochdispersem Siliciumdioxid in wäßriger Aminlösung, insbesondere in 1 ,6-Hexandiamin- oder 1,3-Propandiamin- oder Triethylentetramin-Lösung mit und insbesondere ohne Alkali- oder Erdalkalizusatz zur Reaktion bringt. Auch gehören hierzu die isotaktischen Zeolithe nach EP 34 727 und EP 46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Losung, z.B. 1,6-Hexandiol durchführt.

Den Eisensilikatzeolith erhält man z.B. aus einer Eisenverbindung, vorzugsweise Fe₂(SO₄)₃ und einer Siliciumverbindung, vorzugsweise hochdispersem Siliciumdioxid in wäßriger Aminlösung, insbesondere 1, 6-Hexandiamin, mit oder ohne Alkali- oder Erdalkalizusatz bei 100 bis 200°C unter autogenem Druck.

Zu den verwendbaren siliciumreichen Zeolithen (SiO₂/Al₂O₃ ≧ 10) gehören auch die sog.ZSM-Typen, Ferrierit, NU-1 und Silicalit®, ein Molekularsieb, ein sog. Silica Polymorph.

Die Alumino-, Boro- und Eisensilikatzeolithe können nach ihrer Isolierung, Trocknung bei 100 bis 160°C, vorzugsweise 110°C und Calcinierung bei 450 bis 550°C, vorzugsweise 500°C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem SiO₂/Al₂O₃-Verhältnis von 25:75 bis 90:5, bevorzugt 75:25, Siliciumdioxid, bevorzugt hochdisperses SiO₂, Gemische aus hochdispersem SiO₂ und hochdispersem Al₂O₃, TiO₂, ZrO₂ sowie Ton. Nach der Verformung werden die Extrudate oder Preßlinge bei 110°C/16 h getrocknet und bei 500°C/16 h calciniert.

Man erhält auch vorteilhafte Katalysatoren, wenn der isolierte Aluminobzw. Borosilikatzeolith direkt nach der Trocknung verformt wird und erst nach der Verformung einer Calcinierung unterworfen wird. Die Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegen die Zeolithe aufgrund der Herstellungsweise nicht in der katalytisch aktiven, aciden H-Form vor, sondern z.B. in der Na-Form, dann kann diese durch Ionenaustausch z.B. mit Ammoniumionen und anschließend Calcinierung oder durch Behandlung mit Säuren vollkommen oder partiell in die gewünschte H-Form überführt werden.

Als Hydrierkomponente kann man auf die verformten oder unverformten Zeolithe Übergangsmetalle wie z.B. Metalle der 8. Gruppe wie Fe, Co, Ni, Ru, Rh, Pd, Os, Ir, Pt und der 1. und 2. Nebengruppe wie Cu, Ag, Zn oder deren Gemische aufbringen. Diese Dotierung kann durch Ionenaustausch oder Imprägnierung mit Metallsalzen erfolgen.

Zweckmäßigerweise führt man die Dotierung in einem Steigrohr durch, in dem man die verformten Zeolithe vorlegt und bei 20 bis 100°C eine wäßrige oder ammoniakalische Lösung eines Halogenids oder eines Nitrats der oben beschriebenen Metalle überleitet. Ein derartiger Ionenaustausch kann an der Wasserstoff-, Ammonium- und Alkaliform der Zeolithe vorgenommen werden. Eine weitere Möglichkeit der Metallaufbringung auf die Zeolithe ist gegeben, indem man das zeolithische Material, z.B. mit einem Halogenid, einem Nitrat oder einem Oxid der Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man Cu(NO₃)₂ x 3 H₂O oder Ni(NO₃)2 x 6 H₂O oder Pd(NO₃)₂ in Wasser löst und mit dieser Lösung die verformten oder unverformten Zeolithe eine gewisse Zeit, z.B. 30 Minuten, tränkt. Die eventuell überstehende Lösung wird im Rotationsverdampfer von Wasser befreit. Danach werden die getränkten Zeolithe bei etwa 150°C getrocknet und bei etwa 550°C calciniert. Dieser Tränkvorgang kann mehrmals hintereinander vorgenommen werden, um den gewünschten Metallgehalt einzustellen.

Es ist auch möglich, eine wäßrige Ni(NO₃)₂-Lösung oder ammoniakalische Pd(NO₃)₂-Lösung herzustellen und darin die reinen pulverförmigen Zeolithe bei 40 bis 100°C unter Rühren etwa 24 h aufzuschlämmen. Nach Abfiltrieren, Trocknen bei etwa 150°C und Calcinierung bei etwa 500°C kann das so gewonnene zeolithische Material mit oder ohne Bindemittel zu Strängen, Pellets oder Nirbelgut weiterverarbeitet werden.

Ein Ionenaustausch des in der H-Form oder Ammonium-Form oder Alkali-Form vorliegenden Zeolithen kann in einer Kolonne vorgenommen werden, in der man die Zeolithe in Strängen oder Pellets vorlegt und darüber eine wäßrige Ni(NO₃)₂-Lösung oder ammoniakalische Pd(NO₃)₂-Lösung bei leicht erhöhter Temperatur zwischen 30 und 80°C im Kreislauf 15 bis 20 h leitet. Danach wird mit Wasser ausgewaschen, bei etwa 150°C getrocknet und bei etwa 550°C calciniert. Bei manchen metalldotierten Zeolithen, z.B. Pd-, Cu-, Nidotierten Zeolithen, ist eine Nachbehandlung mit Wasserstoff vorteilhaft.

In manchen Fällen ist es vorteilhaft, den Katalysator vor Reaktionsbeginn zu reduzieren. Beispielsweise ein Pt-, Pd- oder Cu-dotierter ZeolithKatalysator wird im Reaktor auf 170 bis 220°C unter N₂ aufgeheizt, dann wird langsam H₂ zugefügt. Die Temperatur wird so lange konstant gehalten, bis kein H₂O mehr entweicht.

Durch Imprägnierung und Ionenaustausch können gleichzeitig auch mehrere Metalle als Hydrierkomponenten aufgebracht werden.

Wenn bei der Verwendung der zeolithischen Katalysatoren eine durch Koksabscheidung bedingte Desaktivierung eintritt, empfiehlt es sich, die Zeolithe durch Abtrennen der Koksablagerung mit Luft oder mit einem Luft/N₂-Gemisch bei 400 bis 550°C, bevorzugt 500°C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen. Eine gezielte Beeinflussung der Aktivität und Selektivität läßt sich auch durch eine Vorbehandlung z.B. mit H₂S oder einer schwefelhaltigen organischen Verbindung, wie z.B. Mercaptane, Thioharnstoff bei Temperaturen von 150 bis 300°C oder einer schwefelhaltigen anorganischen Verbindung, wie z.B. Dithionit oder Ammoniumsulfid bzw. Ammoniumpolysulfid erreichen. Die Schwefel-Behandlung kann z.B. durch Imprägnierung oder durch Überleiten eines schwefelhaltigen Gases über den Katalysator erfolgen.

Um eine möglichst hohe Selektivität, hohe Umsätze sowie lange Standzeiten zu erreichen, kann es vorteilhaft sein, die Zeolithe zu modifizieren. Eine geeignete Modifizierung der Katalysatoren besteht z.B. darin, daß man zusätzlich zu den oben genannten Hydrierkomponenten den unverformten oder verformten Zeolithen mit Metallsalzen durch einen Ionenaustausch oder durch Imprägnierung dotiert. Als Metalle werden Alkalimetalle wie Li, Cs, K, Erdalkalimetall wie Mg, Ca, Sr, Ba, Metalle der 3., 4. und 5. Hauptgruppe wie B, Al, Ga, Ge, Sn, Pb, Bi, Seltene Erdmetalle wie La, Ce, Pr, Nd, Er, Yb und U eingesetzt. Die Modifizierung mit diesen Metallen kann gleichzeitig mit der Aufbringung der Hydrierkomponente durch Ionenaustausch oder Imprägnierung erfolgen oder sich an die Aufbringung der Hydrierkomponente und eventuell an eine nachfolgende Calcination anschließen.

Zweckmäßigerweise führt man die Dotierung so durch, daß man z.B. den verformten Zeolithen in einem Steigrohr vorlegt und bei 20 bis 100°C z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man Ce(NO₃)₃ x 6 H₂O oder La(NO₃)₂ x 6 H₂O oder Cs₂CO₃ oder H₂WO₃ in Wasser löst. Mit dieser Lösung wird der verformte oder unverformte Zeolith eine gewisse Zeit, ca. 30 Minuten, getränkt. Die eventuell überstehende Lösung wird am Rotationsverdampfer von Wasser befreit. Danach wird der getränkte Zeolith bei ca. 150°C getrocknet und bei ca. 550°C calciniert. Dieser Tränkvorgang kann mehrmals hintereinander vorgenommen werden, um den gewünschten Metallgehalt einzustellen.

Auch ist es möglich, z.B. eine wäßrige Ce(NO₃)₃-Lösung oder Mg(NO₃)₂-Lösung herzustellen und darin den reinen pinlverförmigen Zeolithen bei 40 bis 100°C unter Rühren ca. 24 h aufzuschlämmen. Nach Abfiltrieren, Trocknen bei ca. 150°C und Calcinierung bei ca. 500°C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige Ce(NO₃)₂-Lösung oder Mg(NO₃)₂-Lösung bei leicht erhöhter Temperatur zwischen 30 und 80°C im Kreislauf 15 bis 20 h leitet. Danach wird mit Nasser ausgewaschen, bei ca. 150°C getrocknet und bei ca. 550°C calciniert.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. An diese Art der Modifizierung kann sich dann die Aufbringung der Hydrierkomponente anschließen. Dabei geht man vorteilhaft z.B. so vor, daß man Zeolithe in Pulverform mit 1 n Phosphorsäure 1 Stunde bei 80°C behandelt. Nach der Behandlung wird mit Wasser gewaschen, bei 110°C/16 h getrocknet und bei 500°C/20 h calciniert. Nach einer anderen Arbeitsweise behandelt man Zeolithe vor oder nach ihrer verformung mit Bindemitteln, z.B. 1 bis 3 Stunden bei Temperaturen von 60 bis 80°C mit einer 3 bis 25 gew.%igen, insbesondere 12 bis 20 gew. %igen wäßrigen Salzsäure. Anschließend wird der so behandelte Zeolith mit Wasser gewaschen, getrocknet und bei 400 bis 500°C calciniert. Nunmehr erfolgt die Aufbringung der Hydrierkomponente durch Ionenaustausch bzw. Imprägnierung, wie beschrieben.

Eine besondere Ausführungsform für die Säurebehandlung besteht daß man das zeolithische Material vor seiner Verformung bei erhöhter Temperatur mit 0,001 n bis 2 n, vorzugsweise 0,05 n bis 0,5 n Flußsäure behandelt, beispielsweise durch Erhitzen unter Rückfluß über einen Zeitraum von im allgemeinen 0,5 bis 5, vorzugsweise 1 bis 3 Stunden. Nach Isolierung durch Abfiltrieren und Auswaschen des zeolithischen Materials wird dieses zweckmäßig bei Temperaturen von 100 bis 160°C getrocknet und bei Temperaturen von 450 bis 600°C calciniert. Gemäß einer weiteren bevorzugten Ausführungsform wird das zeolithische Material nach einer Verformung mit Bindemitteln bei erhöhter Temperatur, zweckmäßig bei Temperaturen von 50 bis 90°C, vorzugsweise 60 bis 80°C, über einen Zeitraum von 0,5 bis 5, vorzugsweise mit 12 bis 20 gew.%iger Salzsäure, behandelt. Anschließend wird das zeolithische Material ausgewaschen und zweckmäßig bei Temperaturen von 100 bis 160°C getrocknet und bei Temperaturen von 450 bis 600°C calciniert. Einer HF-Behandlung kann sich auch eine HCl-Behandlung anschließen. Nunmehr erfolgt die Aufbringung der Hydrierkomponenten wie beschrieben.

Nach einer anderen Arbeitsweise kann man Zeolithe durch Aufbringung von Phosphorverbindungen, wie Trimethoxiphosphat, Trimethoxiphosphin, primärem, sekundärem oder tertiärem Natriumphosphat modifizieren. Hierbei werden die Zeolithe in Strang-, Tabletten- oder Wirbelgut-Form z.B. mit wäßriger Phosphat-Lösung getränkt, bei 110°C getrocknet und bei 500°C calciniert. Nunmehr erfolgt die Aufbringung der Hydrierkomponenten, wie voranstehend angeführt.

Weitere Katalysatoren für das erfindungsgemäße Verfahren sind Phosphate, insbesondere Aluminiumphosphate, Siliciumaluminiumphosphate, Siliciumeisenaluminiumphosphate, Cobaltaluminiumphosphat, Cobaltsiliciumaluminiumphosphat, Cerphosphat, Zirkonphosphate, Borphosphat, Eisenphosphate, Strontiumphosphat oder deren Gemische.

Als Aluminiumphosphat-Katalysatoren werden für das erfindungsgemäße Verfahren, insbesondere unter hydrothermalen Bedingungen, synthetisierte Aluminiumphosphate eingesetzt. Geeignete Aluminiumphosphate sind z.B. APO-5, APO-9, APO-11, APO- 12, APO-14, APO-21 , APO-25, APO-31 und APO-33.

Beispielsweise AlPO₄-5 (APO-5) wird synthetisiert, indem man Orthophosphorsäure mit Pseudoboehmit (Catapal SB®) in Wasser homogen mischt; zu dieser Mischung Tetrapropylammoniumhydroxid gibt und danach bei etwa 150°C 20 bis 60 h unter autogenem Druck in einem Autoklaven umsetzt. Das abfiltrierte AlPO₄ wird bei 100 bis 160°C getrocknet und bei 450 bis 550°C calciniert.

AlPO₄-9 (APO-9) wird ebenfalls aus Orthophosphorsäure und Pseudoboehmit aber in wäßriger DABCO-Lösung 4-Diazabicyclo-(2 ,2, 2)octan) bei ca. 200°C unter autogenem Druck während 200 bis 400 h synthetisiert. Verwendet man anstelle DABCO-Lösung Ethylendiamin, so gelangt man zu APO-12.

Die Synthese des AlPO₄-21 (APO-21) erfolgt aus Orthophosphorsäure und Pseudoboehmit in wäßriger Pyrrolidon-Lösung bei 150 bis 200°C unter autogenem Druck während 50 bis 200 h.

Für das Verfahren kann man auch Siliciumaluminiumphosphate wie SAPO-5, SAPO-1 1, SAPO-31 und SAPO-34 einsetzen. Diese Verbindungen kann man durch Kristallisation aus wäßriger Mischung bei 100 bis 250°C und autogenem Druck während 2 h bis 2 Wochen herstellen, wobei die Reaktionsmischung aus einer Silicium-, Aluminium- und Phosphorkomponente in wäßrigen aminoorganischen Lösungen umgesetzt wird.

SAPO-5 beispielsweise wird durch Mischen von SiO₂ suspendiert in wäßriger Tetrapropylammoniumhydroxid-Lösung mit einer wäßrigen Suspension aus Pseudoboehmit und Orthophosphorsäure und anschließende Umsetzung bei 150 bis 200°C, während 20 bis 200 h unter autogenem Druck in einem Rührautoklaven erhalten. Die Trocknung des abfiltrierten Pulvers erfolgt bei 110 bis 160°C und die Calcination bei 450 bis 550°C.

Als Siliciumaluminiumphosphate sind auch ZYT-5, ZYT-6, ZYT-7, ZYT-9, ZYT-11 und ZYT-12 geeignet.

Als Phosphat-Katalysatoren kann man bei dem Verfahren gefällte Aluminiumphosphate einsetzen. Beispielsweise wird ein derartiges Aluminiumphosphat hergestellt, indem 92 g Diammoniumhydrogenphosphat in 700 ml Wasser gelöst werden. Zu dieser Lösung wird 260 g Al(NO₃)₃ x H₂O in 700 ml Wasser über 2 h zugetropft. Dabei wird der pH-Wert durch gleichzeitige Zugabe von 25 %iger NH₃-Lösung bei pH 8 gehalten. Der entstandene Niederschlag wird 12 h nachgerührt, dann abgesaugt, ausgewaschen und bei 60°C/17 h getrocknet.

Geeignete Borphosphate kann man durch Mischen und Kneten von konzentrierter Borsäure und Phosphorsäure und durch anschließende Trocknung und Calcination in Inertgas-, Luft- oder Dampfatmosphäre bei 250 bis 650°C vorzugsweise 300 bis 500°C herstellen.

Auf diese Phosphate werden durch Imprägnierung (Tränken und Aufsprühen) oder in manchen Fällen auch durch [onenaustausch die Hydrierkomponenten, wie beschrieben, aufgebracht. Auch kann wie bei den Zeolithkatalysatoren eine Modifizierung mit Metallen oder Säuren erfolgen.

Die Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Die katalytische Umwandlung wird bevorzugt in der Gasphase bei 100 bis 500°C, vorzugsweise 200 bis 400°C, und einer Belastung WHSV = 0,1 bis 20 h⁻¹, bevorzugt 0,5 bis 5 h⁻¹ (g Ausgangsstoff/g Katalysator und Stunde) durchgeführt.

Das Verhältnis Wasserstoff zu Epoxid ist zweckmäßig 1-100 molar, insbesondere 3-30 molar.

Die Gasphasenreaktion kann in einem Festbett oder in einem Wirbelbett ausgeführt werden.

Es ist auch möglich, die Reaktion in der Flüssigphase (Suspension-, Riesel- oder Sumpffahrweise) bei Temperaturen zwischen 50 und 200°C durchzuführen.

Das Verfahren kann bei Normaldruck, vermindertem oder erhöhtem Druck durchgeführt, diskontinuierlich oder vorzugsweise kontinuierlich erfolgen.

Schwerflüchtige oder feste Ausgangsstoffe werden in gelöster Form, z.B. in THF-, Toluol- oder Petrolether-Lösung eingesetzt. Allgemein ist eine Verdünnung des Ausgangsstoffes mit derartigen Lösungsmitteln oder mit Inertgasen wie N₂, Ar, H₂O-Dampf möglich.

Nach der Umsetzung werden die entstandenen Produkte durch übliche Verfahren, z.B. durch Destillation aus dem Reaktionsgemisch isoliert; nichtumgesetzte Ausgangsstoffe werden gegebenenfalls in die Umsetzung zurückgeführt.

Bevorzugt werden die gasförmigen Reaktionsprodukte sofort in eine Trennung eingebracht und anschließend in ihre Einzelkomponenten zerlegt. Eine derartige Trennung kann z.B. in einer Fraktionierkolonne durchgeführt werden.

### Beispiele 1 bis 15

Die Reaktionen in der Gasphase werden unter isothermen Bedingungen in einem Rohrreaktor (Wendel, 0,6 cm Innendurchmesser, 90 cm Länge) mindestens 6 Stunden lang durchgeführt. Die Reaktionsprodukte wurden durch übliche Methoden abgetrennt und charakterisiert. Die quantitative Bestimmung der Reaktionsprodukte und der Ausgangsprodukte erfolgt gaschromatographisch.

Die für das erfindungsgemäße Verfahren eingesetzten Katalysatoren sind:

### Katalysator A

Der Borosilikatzeolith des Pentasil-Typs wird in einer hydrothermalen Synthese aus 640 g hochdispersem SiO₂, 122 g H₃BO₃, 8000 g einer wäßrigen 1. 6-Hexandiamin-Lösung (Mischung 50:50 Gew.") bei 170°C unter autogenem Druck in einem Rührautoklaven hergestellt. Nach Abfiltrieren und Auswaschen wird das kristalline Reaktionsprodukt bei 100°C/24 h getrocknet und bei 500°C/24 h calciniert. Dieser Borosilikatzeolith setzt sich zusammen aus 94,2 Gew.% SiO₂ und 2,3 Gew.% B₂O₃.

Mit diesem Material werden durch Verformen mit einem Verformungshilfsmittel 2-mm-Stränge hergestellt, die bei 110°C/16 h getrocknet und bei 500°C/124 h calciniert werden.

Katalysator A erhält man, indem man diese Stränge mit einer wäßrigen Cu(NO₃)₂-Lösung imprägniert, danach bei 130°C/2 h trocknet und bei 540°C/2 h calciniert. Der Cu-Gehalt beträgt 3,4 Gew.%.

### Katalysator B

Katalysator B wird wie Katalysator A hergestellt, jedoch mit wäßriger Pd(NO₃)₂-Lösung statt Cu(NO₃)₂-Lösung getränkt. Der Pd-Gehalt beträgt 0,5 Gew.%.

### Katalysator C

Die Stränge des Borosilikatzeolithen, wie bei Katalysator A beschrieben, werden in einer Kolonne vorgelegt und einem Ionenaustausch mit einer ammoniakalischen Palladiumnitrat-Lösung bei 50°C unterworfen. Nach Auswaschen mit H₂O wird bei 110°C getrocknet und bei 500°C/5 h calciniert. Der Pd-Gehalt betragt 0,95 Gew.%.

### Katalysator D

Katalysator D wird wie Katalysator A hergestellt, jedoch mit einer wäßrigen Lösung aus Pd- und Ce-Nitrat anstatt Cu-Nitrat getränkt. Der Pd-Gehalt beträgt 0,5 Gew.%. der Ce-Gehalt 2,3 Gew.%.

### Katalysator E

Katalysator E wird erhalten, indem man Katalysator C mit Ammonsulfid-Lösung imprägniert. Der S-Gehalt beträgt 0,35 Gew.%.

### Katalysator F

Aluminosilikatzeolith vom Pentasil-Typ wird unter hydrothermalen Bedingungen bei autogenem Druck und 150°C aus 65 g hochdispersem SiO₂, 20,3 g Al₂(SO₄)₃ x 18 H₂O in 1 kg einer wäßrigen 1 ,6-Hexandiamin-Lösung (Mischung 50:50 Gew.%) in einem Rührautoklaven hergestellt, Nach Abfiltrieren und Auswaschen wird das kristalline Reaktionsprodukt bei 100°C/24 h getrocknet und bei 500°C/24 h calciniert. Dieser Aluminosilikatzeolith enthält 91,6 Gew.% SiO₂ und 4,6 Gew.% Al₂O₃.

Der Katalysator wird mit einem Verformungshilfsmittel zu 2-mm-Strängen verformt, bei 110°C16 h getrocknet und bei 500°C/24 h calciniert.

Katalysator F erhält man, indem man diese Stränge mit einer wäßrigen Cu(NO₃)₂-Lösung imprägniert, danach bei 130°C/2 h trocknet und bei 540°C/2 h calciniert. Der Cu-Gehalt beträgt 3,0 Gew.%.

### Katalysator G

Siliciumaluminiumphosphat-5 (SAPO-5) wird aus einer Mischung aus 200 g 98 %iger Phosphorsäure, 136 g Boehmit, 60 g Kieselsol (30 %ig), 287 g Tripropylamin und 587 g H₂O hergestellt. Diese Mischung wird bei 150°C während 168 Stunden unter autogenem Druck umgesetzt. Nach Filtration wird das kristalline Produkt bei 120°C getrocknet und bei 500°C calciniert.

SAPO-5 enthält 49,8 Gew.% P₂O₅, 33,0 Gew.% Al₂O₃, 6,2 Gew.% SiO₂. SAPO-5 wird mit einem Verstrangungsmittel zu 3-mm-Strängen verformt, bei 120°C getrocknet und bei 500°C calciniert.

Diese Stränge werden mit einer ammoniakalischen Palladiumnitrat-Lösung imprägniert, Nach Auswaschen mit H₂O wird bei 110°C getrocknet und bei 500°C/5 h calciniert. Der Pd-Gehalt beträgt 1 Gew.%.

Die mit diesen Katalysatoren erzielten Versuchsergebnisse und Versuchsbedingungen sind in der Tabelle zusammengefaßt.

### Beispiel 16

Es werden 200 ml/h Styroloxid in einem Wasserstoffstrom von 400 1/h verdampft und bei 260°C über 1 l des Katalysators A, der in einem von außen elektrisch beheizten Reaktionsrohr untergebracht ist, geleitet. Die gasförmigen Reaktionsprodukte werden kondensiert und nach üblichen Methoden aufgearbeitet und charakterisiert. Die Reinherstellung des Phenylethanols gelingt durch eine übliche Destillation. Es werden 78,4 % Destillationsausbeute an Phenylethanol isoliert.

**Tabelle 1**

| Herstellung von Phenylethanol (I) aus Styroloxid (II) | | | | | | | | | |
|---|---|---|---|---|---|---|---|---|---|
| Beispiel | 1 | 2 | 3 | 4 | 5 | 6 | 7 | 8 | 9 |
| Katalysator | A | A | A | B | C | D | E | F | G |
| Temperatur [°C] | 300 | 300 | 250 | 250 | 250 | 250 | 250 | 250 | 300 |
| WHSV [h-¹]¹⁾ | 3,0 | 2,5 | 1,5 | 1,5 | 2,5 | 2,5 | 1,5 | 1,5 | 1,5 |
| 1 H₂/h | 3 | 7 | 15 | 7 | 7 | 7 | 7 | 15 | 7 |
| Umsatz von (II) [%] | 100 | 100 | 100 | 100 | 100 | 100 | 100 | 100 | 100 |
| Selektivität [%] von (I) | 34,2 | 52,2 | 84,7 | 72,7 | 75,2 | 80,4 | 83,9 | 82,4 | 70,3 |
| Selektivität [%] von (III)²⁾ | 49,3 | 22,9 | - | - | - | - | - | - | - |

| | | | | | | | | | |
|---|---|---|---|---|---|---|---|---|---|
| ¹⁾ bezogen auf Zulauf des Styroloxids (11) | | | | | | | | | |
| ²⁾ Phenylacetaldehyd (III) | | | | | | | | | |
| ³⁾ Nebenprodukte sind z.B. Styrol, Ethylbenzol, Toluol, Xylol | | | | | | | | | |

**Tabelle 2**

| Herstellung von substituierten Phenylethanolen (I) aus entsprechenden Styroloxiden (II) | | | | | | |
|---|---|---|---|---|---|---|
| Beispiel | 10 | 11 | 12 | 13 | 14 | 15 |
| Einsatzstoff | 4-Methyl- | 4-Fluor- | 4-Trifluor-methyl- | 4-Methoxy- | 2-Methyl-4-fluor- | 2-Methyl- |
| Katalysator | A | A | A | A | A | A |
| Temperatur [°C] | 250 | 250 | 250 | 250 | 250 | 250 |
| WHSV [h⁻¹] | 1,5 | 1,5 | 1,5 | 1,5 | 1,5 | 1,5 |
| 1 H₂/h | 15 | 15 | 15 | 15 | 15 | 15 |
| Umsatz (I) [%] | 100 | 100 | 100 | 100 | 100 | 100 |
| Selektivität(II) [%] | 90,2 | 81,5 | 85,2 | 87,4 | 75,3 | 88,7 |
| WHSV ist bezogan auf Zulauf des Styraloxids | | | | | | |

## Patentansprüche

1. Verfahren zur Herstellung von Phenylethanolen der Formel (I) 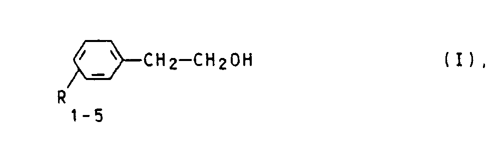 wobei R Wasserstoff, Alkyl- mit 1 bis 4 Kohlenstoffatomen, Alkoxy- mit 1 bis 4 Kohlenstoffatomen, Fluor-, Trifluormethyl- und/oder Trifluormethoxy-Reste bedeuten, dadurch gekennzeichnet, daß man Epoxide der Formel (II) 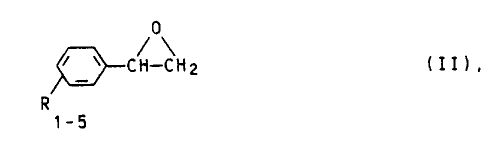 wobei R obige Bedeutung besitzt, in Gegenwart von Wasserstoff an Zeolithen in der aciden H-Form und/oder Phosphaten, die mit Hydriermetallen dotiert sind, als Katalysatoren umsetzt.

2. Verfahren nach Anspruch 1, dadurch gekennzeichnet, daß man Zeolithe des Pentasiltyps verwendet.

3. Verfahren nach Anspruch 1, dadurch gekennzeichnet, daß man Aluminium-, Bor- und/oder Eisensilikatzeolithe des Pentasiltyps verwendet.

4. Verfahren nach Anspruch 1, dadurch gekennzeichnet, daß man Zeolithe des Faujasit-, Mordenit-, Erionit-, Chabazit- und/oder L-Typs verwendet.

5. Verfahren nach Anspruch 1, dadurch gekennzeichnet daß man Phosphate der Elemente Al, B, Fe, Zr, Ce verwendet.

6. Verfahren nach Anspruch 1, dadurch gekennzeichnet. daß man hydrothermal hergestellte Phosphate verwendet.

7. Verfahren nach Anspruch dadurch gekennzeichnet, daß man Siliciumaluminiumphosphate, Siliciumborphosphate, Eisensiliciumaluminiumphosphate, Siliciumeisenphosphate, Cobaltaluminiumphosphate und/oder Cobaltsiliciumaluminiumphosphate verwendet.

8. Verfahren nach Anspruch 1, dadurch gekennzeichnet, daß man als Hydriermetalle Metalle aus der Pt-Gruppe, Ag, Co, Cu, Zn und deren Gemische verwendet.

9. Verfahren nach Anspruch 1, dadurch gekennzeichnet, daß man die Reaktion in der Gasphase durchführt.

## Claims

1. A process for preparing a phenylethanol of the formula (I) 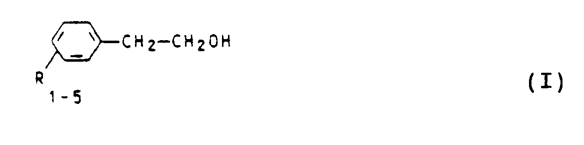 where R is hydrogen, alkyl of from 1 to 4 carbon atoms, alkoxy of from 1 to 4 carbon atoms, fluorine, trifluoromethyl or trifluoromethoxy, which comprises reacting an epoxy of formula (II) 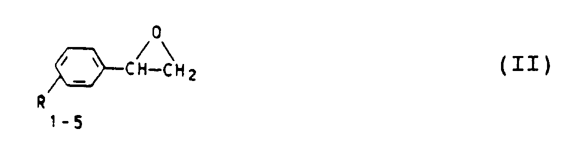 where R is as defined above, in the presence of hydrogen over a catalyst comprising a zeolite in the acidic H-form or a phosphate, each doped with a hydrogenating metal.

2. A process as claimed in claim 1, wherein the zeolite used is of the pentasil type.

3. A process as claimed in claim 1, wherein an aluminosiltcate, borosilicate or iron silicate zeolite of the pentasil type is used.

4. A process as claimed in claim 1, wherein the zeolite used is of the faujasite, mordenite, erionite, chabazite or L-type.

5. A process as claimed in claim 1, wherein a phosphate of the elements Al, B, Fe, Zr or Ce is used.

6. A process as claimed in claim 1, wherein a hydrothermally prepared phosphate is used.

7. A process as claimed in claim 1, wherein a silicon aluminum phosphate, silicon boron phosphate, iron silicon aluminum phosphate, silicon iron phosphate, cobalt aluminum phosphate or cobalt silicon aluminum phosphate is used.

8. A process as claimed in claim 1, wherein the hydrogenating metal used is a metal of the Pt group, Ag, Ni, Co, Cu, Zn or a mixture thereof.

9. A process as claimed in claim 1, wherein the reaction is carried out in the gas phase.

## Revendications

1. Procédé de fabrication de phényléthanols de la formule (I) 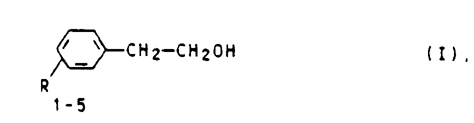 dans laquelle R représente un atome d'hydrogène, un radical alkyle qui comporte de 1 à 4 atomes de carbone, un radical alcoxy qui comporte de 1 à 4 atomes de carbone, un atome de fluor, un radical trifluorométhyle et/ou trifluorométhoxy, caractérisé en ce que l'on convertit des époxydes de la formule (II) 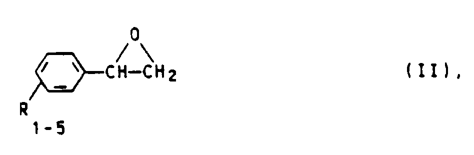 dans laquelle R possède les significations qui lui ont été attribuées ci-dessus, en présence d'hydrogène, sur des zéolites, en la forme H acide et/ou en phosphates, qui sont dopés à l'aide de métaux d'hydrogénation.

2. Procédé suivant la revendication 1, caractérisé en ce que l'on utilise des zéolites du type pentasile.

3. Procédé suivant la revendication 1, caractérisé en ce que l'on emploie des zéolites du type alumino-, boroet/ou sidérosilicate du type pentasile.

4. Procédé suivant la revendication 1, caractérisé en ce que l'on emploie des zéolites du type faujasite, mordénite, érionite, chabazite et/ou du type L.

5. Procédé suivant la revendication 1, caractérisé en ce que l'on utilise des phosphatesdes éléments Al, B, Fe, Zr, Ce,

6. Procédé suivant la revendication 1, caractérisé en ce que l'on utilise des phosphates préparés par voie hydrothermique.

7. Procédé suivant la revendication 1, caractérisé en ce que l'on utilise des phosphates de silicium-aluminium, des phosphates de silicium-bore, des phosphates de fer, de silicium et d'aluminium, des phosphates de silicium et de fer, des phosphates de cobalt et d'aluminium et/ou des phosphates de cobalt, de silicium et d'aluminium.

8. Procédé suivant la revendication 1, caractérisé en ce que l'on utilise des métaux du groupe du platine, Ag, Ni, Co, Cu, Zn et leurs mélanges, à titre de métaux d'hydrogénation.

9. Procédé suivant la revendication 1, caractérisé en ce que l'on entreprend la réaction en phase gazeuse.
